# EUROPEAN PATENT APPLICATION

(11) **EP 3 175 867 A1**
(43) Date of publication of application: **07.06.2017**
(21) Application number: 15197882.2
(22) Date of filing: 03.12.2015
(51) Int. Cl.: A61L 2/232, A47J 31/44, A01N 25/10, A01N 59/16, A61L 2/238, C02F 1/72, C09D 5/16, D06F 35/00, F24C 15/00, F24C 15/20, H05B 6/64, A47L 15/42, A61L 2/235, A61L 9/18, C08K 5/00, C08K 5/56, C02F 103/00

(54) **USE OF (HETERO)POLYOXOMETALATES FOR SIMULTANEOUSLY IMPARTING ANTIMICROBIAL PROPERTIES TO A SURFACE OF A SUBSTRATE AND REDUCING THE GROWTH OF A BIOFILM ON THE SURFACE OF THE SUBSTRATE**

(71) Applicant: POM Patentverwaltungs GbR, 35619 Braunfels (DE)
(72) Inventor: SCHEPERS, Klaus, 35619 Braunfels (DE); MISCHO, Horst, 54295 Trier (DE); BIRKEL, Alexander, 64287 Darmstadt (DE)
(74) Representative: Teipel, Stephan

(57) **Abstract**

The present invention relates to the use of at least one (hetero)polyoxometalate for simultaneously imparting antimicrobial properties to a surface of a substrate and reducing the growth of a biofilm on the surface of the substrate. Furthermore, the present invention relates to a substrate comprising a (hetero)polyoxometalate or a mixture of (hetero)polyoxometalates, wherein the substrate is selected from a polymer body, paint, varnish and coating.

## Description

The present invention relates to the use of at least one (hetero)polyoxometalate for simultaneously imparting antimicrobial properties to a surface of a substrate and reducing the growth of a biofilm on the surface of the substrate.

The use of polyoxometalates is known in the art for several purposes, e.g. in the general area of analytical chemistry (e.g. elemental analysis, electron staining), the use as catalysts including photo catalysts, in biochemistry for inhibiting electron transfer processes and as electron-dense and rigid components in the crystallization of biomolecules (e.g. ribosomes, leading to the 2009 Nobel Prize), and in medicine due to their antiviral and antitumor activity. The use of polyoxometalates as acid and oxidation catalysts is known in industry (e.g. for the hydration of olefins). Furthermore, (hetero)polyoxometalates are used as delignification agents in bleaching.

EP 2 765 136 A1 and WO 2014/122225 A1 disclose the use of heteropolyoxometalates for antimicrobial and disinfecting purposes in substrates, paints and coatings. The (hetero)polyoxometalates described therein consist of a (hetero)polyoxometalate anion and a cation, which is selected from quaternary ammonium cations, quaternary phosphonium cations and tertiary sulfonium cations. The cation makes the heteropolyoxometalate more stable, in particular with respect to its thermal stability.

However, while the heteropolyoxometalates described in EP 2 765 136 A1 and WO 2014/122225 A1 show antimicrobial activity, these heteropolyoxometalates are not necessarily useful in the long term reduction of the biofilm growth on a substrate surface.

Accordingly, there is still a need for (hetero)polyoxometalates that are useful in providing antimicrobial properties to the surface of a substrate as well as in the long term reduction, preferably in the long term inhibition, of biofilm growth on the surface of a substrate.

It has now been found that certain (hetero)polyoxometalates are surprisingly useful for both, i.e. for simultaneously imparting antimicrobial properties to a surface of a substrate and reducing, preferably inhibiting, on a long term time scale the growth of a biofilm on the surface of the substrate. A long term time scale means in the present context at least 2 weeks, preferably at least 1 month, more preferably at least 1 year, and even more preferably at least 3 years.

The present invention therefore relates to the use of at least one (hetero)polyoxometalate for simultaneously imparting antimicrobial properties to a surface of a substrate and reducing the growth of a biofilm on the surface of the substrate, wherein the at least one (hetero)polyoxometalate is of the formula **(I)**, **(II)**, **(III)**, **(IV)** and/or **(V)**:

**[Aₙ]^{m+}[XM_{q}Z_{r-q-o}Z'ₒOₛ]^{m-}** **(I),**

wherein
m- is the negative charge of the (hetero)polyoxometalate anion,
m+ is the positive charge of the cation(s) A,
|m-| = |m+|
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
r = 11 or 12,
o = 0 or 1,
s = 37, 38, 39 or 40,
when r = 11, q = 0, 1, 2, 3, 4, 5, 6, 7, 8 or 9,
when r = 12, q = 0, 1, 2, 3, 4, 5, 6, 7, 8, 9 or 10, and
when r = 12, s = 40, X = P and q is 0, 1, 2 or 3, M is not V;

**[Aₙ]^{m+}[XM_{q}Z_{t-q-o}Z'ₒO₂₄]^{m-}** **(II)**,

wherein
m- is the negative charge of the (hetero)polyoxometalate anion,
m+ is the positive charge of the cation(s) A,
|m-| = |m+|
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
t = 4 or 6,
o = 0 or 1,
when t = 6, q = 0, 1, 2, 3 or 4,
when t = 4, q = 0, 1 or 2, and
when t = 4 and X = P, q is 1, 2 or 3;

**[Aₙ]^{m+}[XₚM_{q}Z_{6-q-p-o}Z'ₒO₁₉]^{m-}** **(III)**,

wherein
m- is the negative charge of the (hetero)polyoxometalate anion,
m+ is the positive charge of the cation(s) A,
|m-| = |m+|
n is the number of cation(s) A required to provide the positive charge m+, Z and Z' are independently selected from W and Mo,
X is selected from P, Si, Ge, Al and B,
M is selected from Ti, V, Mn, Fe, Co, Ni, Zn and Cr,
o = 0 or 1,
p = 0 or 1, and
q = 0, 1, 2 or 3
when p = 0, M is not V;

**[A**ₙ**]**^{m+}**[X**₂**M**_{q}**Z**_{18-q-o}**Z'**ₒ**O**₆₂**]**^{m-} (IV),

wherein
m- is the negative charge of the (hetero)polyoxometalate anion,
m+ is the positive charge of the cation(s) A,
|m-| = |m+|
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
o = 0 or 1,
q = 0, 1, 2, 3, 4, 5, 6, 7, 8, 9, 10, 11, 12, 13, 14, 15 or 16, and
when X = P, q is 1, 2 or 3;
**[Aₙ]^{m+}[X₅M_{q}Z_{30-q-o}Z'ₒO₁₁₀]^{m-}** (V),

wherein
m- is the negative charge of the (hetero)polyoxometalate anion,
m+ is the positive charge of the cation(s) A,
|m-| = |m+|
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
o = 0 or 1,
q = 0, 1, 2, 3, 4, 5, 6, 7, 8, 9, 10, 11, 12, 13 ,14, 15, 16, 17, 18, 19, 20, 21, 22, 23, 24, 25, 26 or 27;
wherein A is selected from one or more cations and comprises at least one cation selected from the group consisting of quaternary cations, quaternary phosphonium cations and tertiary sulfonium cations,
with the proviso that the (hetero)polyoxometalate is not [(CH₃(CH₂)₅)₄N]₇[SiV₃W₉O₄₀].

The present invention furthermore relates to a substrate comprising a (hetero)polyoxometalate as defined herein, or a mixture thereof, wherein the substrate is selected from the group consisting of a polymer body, a paint, a varnish, a coating, an ink, glas fibers or an inorganic oxide material.

Preferred embodiments of the present invention are defined in the subclaims.

### Description of the drawings

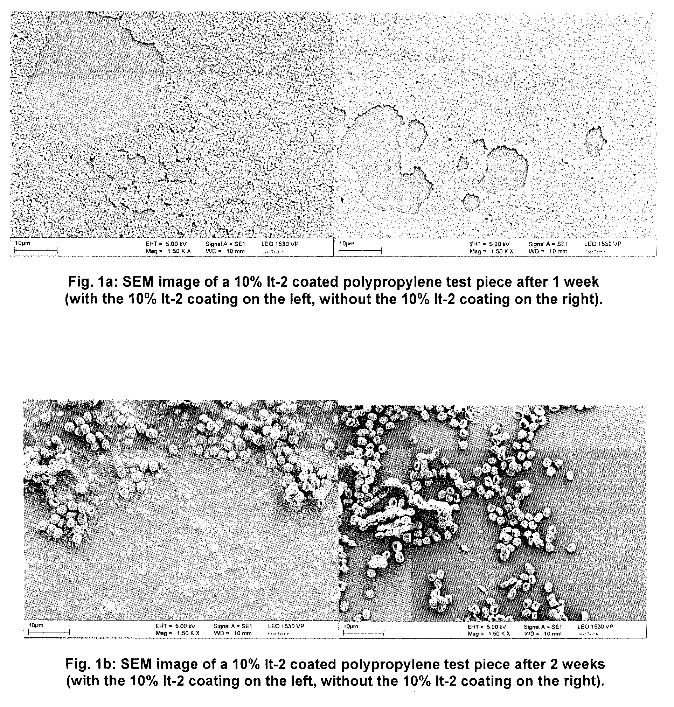

**Fig. 1** shows the Staphylococcus aureus biofilm development on a polypropylene test piece coated on one part with an active coating comprising 10% of the heteropolyoxometalate [(C₄H₉)₃P(C₁₄H₂₉)]₇[SiV₃W₉O₄₀] (It-2), compared to the uncoated part of the polypropylene piece after 1 week (**Fig. 1a**) and after 2 weeks (**Fig. 1b**), as determined by SEM spectroscopy.

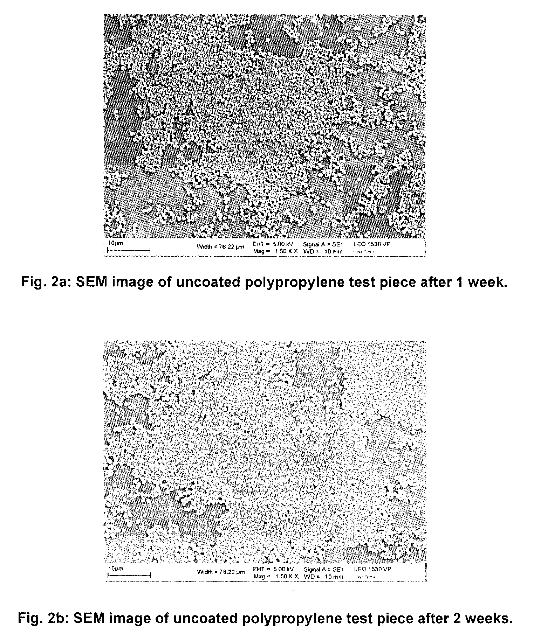

**Fig. 2** shows the Staphylococcus aureus biofilm development on a polypropylene piece without (hetero)polyoxometalate-functionalized active coating (blind sample) after 1 week (**Fig. 2a**) and after 2 weeks (**Fig. 2b**), as determined by SEM spectroscopy.

### Detailed description

The (hetero)polyoxometalates described herein surprisingly show antimicrobial properties and simultaneously reduce the growth of a biofilm on the surface of a substrate. The substrate referred to herein is typically a polymer body, a paint, a varnish, a coating, an ink, glass fibers or an inorganic oxide material, wherein the (hetero)polyoxometalate as described herein is incorporated. The antimicrobial (hetero)polyoxometalates described herein are particularly useful in the reduction, preferably in the inhibition, of the growth of a bacteria biofilm on the surface of a substrate.

The (hetero)polyoxometalates as described herein consist of a cationic moiety [Aₙ]^{m+}, which imparts antimicrobial activity to the compound, and the (hetero)polyoxometalate anion. The (hetero)polyoxometalates of the present invention show a flexible redox behavior, which means that they can be reversibly reduced by one or more electrons. In particular, it has been found that these (hetero)polyoxometalates, when incorporated into a substrate, have the ability to activate molecular oxygen and/or hydrogen peroxide, preferably molecular oxygen, which means that an electron is transferred from the (hetero)polyoxometalate to molecular oxygen and/or hydrogen peroxide, preferably molecular oxygen, resulting in the formation of reactive oxygen species (ROS) such as the hyperoxide anion (O₂⁻) and an oxidized (hetero)polyoxometalate species.

The reactive oxygen species formed by (hetero)polyoxometalate-induced activation of molecular oxygen and/or hydrogen peroxide, preferably of molecular oxygen, reduces the growth of a biofilm on a surface of a substrate comprising the (hetero)polyoxometalate. This, in turn, is useful for maintaining the microbial activity of the substrate resulting from the at least one (hetero)polyoxometalate comprised therein on a long term time scale. A long term time scale, as used in the context of the present invention, means at least 2 weeks, preferably at least 1 month, more preferably at least 1 year, even more preferably at least 3 years.

The cationic moiety of the oxidized (hetero)polyoxometalate species then reacts with the negatively charged cell membrane of a microorganism thereby killing the microorganism, resulting in an antimicrobial activity of the (hetero)polyoxometalate and the substrate comprising the (hetero)polyoxometalate, respectively.

The (hetero)polyoxometalates described herein thus provide a synergistic effect: They have antimicrobial properties because of their ability to react with the cell membranes of microorganisms, and they have the ability to generate reactive oxygen species (ROS), in particular the hyperoxide anion (O₂⁻), that reduces the growth of a biofilm on the surface of the substrate comprising the (hetero)polyoxometalate, with the result that the antimicrobial activity of the substrate is maintained on a long term time scale (at least 2 weeks, preferably at least 1 month, more preferably at least 1 year, even more preferably at least 3 years).

The (hetero)polyoxometalates described herein thus represent heterogeneous catalysts which, because of their flexible redox behavior, regenerate themselves by the reaction with molecular oxygen and/or hydrogen peroxide, preferably molecular oxygen, thereby allowing a long term efficacy of the substrate comprising the (hetero)polyoxometalate against microorganisms for at least 2 weeks, preferably at least 1 month, more preferably at least 1 year, even more preferably at least 3 years.

The (hetero)polyoxometalates described herein can also be used for providing self-cleaning, stripping, disinfecting, self-sanitizing, biocidal, antimicrobial, and/or deodorizing properties to at least part of a substrate or a surface of a substrate, and/or for the decomposition and/or degradation of organic materials on a surface of a substrate or in the vicinity thereof.

The term "(hetero)polyoxometalate", as used herein, encompasses the (hetero)polyoxometalates of formulae **(I)**, **(II)**, **(III)**, **(IV)** and **(V)** (and sub-formulae thereof as described herein).

In the heteropolyoxometalates described herein, the "heteroatom" is phosphorus, silicon, germanium, aluminum or boron. If phosphorus, silicon, germanium, aluminum or boron are not included, the term "polyoxometalate" is appropriate for the designation of the species.

All (hetero)polyoxometalates described herein have molybdenum or tungsten oxide subunits which can be replaced in parts by titanium, vanadium, manganese, iron, cobalt, nickel or chromium oxide subunits.

The term "antimicrobial properties", as used herein, refers to the ability of the (hetero)polyoxometalates of formulae **(I)**, **(II)**, **(III)**, **(IV)** and **(V)** (and sub-formulae thereof as described herein) to kill microorganisms.

The term "microorganisms", as referred to herein, include bacteria, viruses, fungi and algae. Preferably, the term "microorganisms", as used herein, refers to bacteria.

The term "biofilm", as used herein, refers to an assembly of microorganisms wherein cells stick to each other on the surface of a substrate. The term "growth of biofilm" or "biofilm growth", as used herein, refers to the microorganism built-up adhering to a surface of a substrate.

The term "reduction of the growth of a biofilm", as used herein, refers to the decrease, preferably the inhibition, of the microorganism built-up on a long term scale, i.e. at least 2 weeks, preferably at least 1 month, more preferably at least 1 year, even more preferably at least 3 years, on the surface of a substrate comprising a (hetero)polyoxometalate as described herein compared to the surface of a substrate not comprising a (hetero)polyoxometalate as described herein.

The abbreviation "wt.-%" or "w/w", as used herein, means "weight percentage" and refers to the weight amount of a compound in relation to the (total) weight of a composition of compounds or of a substrate if nothing else is explicitly stated or obvious under the circumstances.

It is understood that the cation(s) A is/are selected such that in the (hetero)polyoxometalate the charge of the compound of the Formula **(I)**, **(II)**, **(III)**, **(IV)** and **(V)** is zero. For achieving this either the number and/or charge of cation(s) A can be altered and/or the charge is balanced by one or more further cations and/or anions.

Generally, polyoxometalates based on molybdenum (Mo) or tungsten (W) are known in the art, in particular as Keggin-type heteropolyoxometalate anions [XZ₁₂O₄₀]ⁿ⁻ wherein the central heteroatom (X) can be e.g. phosphorus (P⁵⁺), silicon (Si⁴⁺), germanium (Ge⁴⁺), aluminum (Al³⁺), boron (B³⁺) etc.. Further, in the Keggin-type polyoxometalate based on molybdenum or tungsten a number (1 or more) of molybdenum or tungsten atoms can be replaced by titanium atoms (e.g. Ti⁴⁺), vanadium atoms (e.g. V⁵⁺), nickel atoms (e.g. Ni²⁺), iron atoms (e.g. Fe³⁺), cobalt atoms (e.g. Co²⁺ or Co³⁺), zinc atoms (Zn²⁺), chromium atoms (e.g. Cr²⁺ or Cr³⁺), manganese atoms (e.g. Mn²⁺) etc.

Another example of polyoxometalate anions is the Wells-Dawson species [X₂Z₁₈O₆₂]ⁿ⁻. This heteropolyoxometalate anion contains two heteroatoms (X), which can e.g. be phosphorus (P⁵⁺), silicon (Si⁴⁺), germanium (Ge⁴⁺), aluminum (Al³⁺), boron (B³⁺) and 18 molybdenum or tungsten atoms (Z). Further, in the Wells-Dawson-type polyoxometalate based on tungsten one or more tungsten atoms may be replaced e.g. by vanadium atoms (e.g. V⁵⁺).

Further well-known structures are the Venturello structure [XZ₄O₂₄]ⁿ and the Anderson structure [XZ₆O₂₄]ⁿ⁻ with Z being tungsten (W⁶⁺) or molybdenum (Mo⁶⁺) and X being a heteroatom such as phosphorus (P⁺⁵).

Structures of the formulae [Z₆O₁₉]ⁿ⁻ are known as Lindquist structures with Z being (W⁶⁺) or molybdenum (Mo⁶⁺).

(NH₄)₁₄Na[P₅W₃₀O₁₀₀] is known as the Preyssler-type heteropolyoxometalate.

Generally, these (hetero)polyoxometalate anions are known in the art, and the negative charge of the known anions is typically counterbalanced by cations like Li⁺, Na⁺, K⁺ or NH₄⁺ which provide solubility of the resulting salts in water.

In a preferred embodiment, the present invention relates to the use as described herein of (hetero)polyoxometalates of formulae **(I)**, **(II)**, **(III)**, **(IV)** and **(V)**, wherein in formulae **(I)**, **(II)**, **(III)**, **(IV)** and **(V) Z** = W. With o = 0, this embodiment covers (hetero)polyoxometalates of the following formulae **(I')**, **(II')**, **(III')**, **(IV')** and **(V')**:

**[Aₙ]^{m+}[XM_{q}W_{r-q}Oₛ]^{m-}** **(I')**,

wherein
m- is the negative charge of the heteropolyoxometalate anion,
m+ is the positive charge of the cation(s) A,
|m-| = |m+|
n is the number of cation(s) A required to provide the positive charge m+,
X is selected from P, Si, Ge, Al and B,
M is selected from Ti, V, Mn, Fe, Co, Ni, Zn and Cr,
r = 11 or 12,
s = 37, 38, 39 or 40,
when r = 11, q = 0, 1, 2, 3, 4, 5, 6, 7, 8 or 9,
when r = 12, q = 0, 1, 2, 3, 4, 5, 6, 7, 8, 9 or 10, and
when r = 12, s = 40, X = P and q is 0, 1, 2 or 3, M is not V;

**[A**ₙ**]**^{m}**⁺[XM**_{q}**W**_{t-q}**O**₂₄**]**^{m**-**} **(II')**,

wherein
m- is the negative charge of the heteropolyoxometalate anion,
m+ is the positive charge of the cation(s) A,
|m-| = |m+|
n is the number of cation(s) A required to provide the positive charge m+,
X is selected from P, Si, Ge, Al and B,
M is selected from Ti, V, Mn, Fe, Co, Ni, Zn and Cr,
t = 4 or 6,
when t = 6, q = 0, 1, 2, 3 or 4,
when t = 4, q = 0, 1 or 2, and
when t = 4 and X = P, q is 1, 2 or 3;

**[A**ₙ**]**^{m}**⁺[X**ₚ**M**_{q}**W**_{6-q-p}**O**₁₉**]**^{m-} **(III')**,

wherein
m- is the negative charge of the (hetero)polyoxometalate anion,
m+ is the positive charge of the cation(s) A,
|m-| = |m+|
n is the number of cation(s) A required to provide the positive charge m+,
X is selected from P, Si, Ge, Al and B,
M is selected from Ti, V, Mn, Fe, Co, Ni, Zn and Cr,
p = 0 or 1, and
q = 0, 1, 2 or 3
when p = 0, M is not V;

**[Aₙ]^{m+}[X₂M_{q}W_{18-q}O₆₂]^{m-}** **(IV')**,

wherein
m- is the negative charge of the heteropolyoxometalate anion,
m+ is the positive charge of the cation(s) A,
|m-| = |m+|
n is the number of cation(s) A required to provide the positive charge m+,
X is selected from P, Si, Ge, Al and B,
M is selected from Ti, V, Mn, Fe, Co, Ni, Zn and Cr,
q = 0, 1, 2, 3, 4, 5, 6, 7, 8, 9, 10, 11, 12, 13, 14, 15 or 16, and
when X = P, q is 1, 2 or 3;

**[Aₙ]^{m+}[X₅M_{q}W_{30-q}O₁₁₀]^{m-}** **(V'),**

wherein
m- is the negative charge of the heteropolyoxometalate anion,
m+ is the positive charge of the cation(s) A,
|m-| = |m+|
n is the number of cation(s) A required to provide the positive charge m+,
X is selected from P, Si, Ge, Al and B,
M is selected from Ti, V, Mn, Fe, Co, Ni, Zn and Cr,
q = 0, 1, 2, 3, 4, 5, 6, 7, 8, 9, 10, 11, 12, 13 ,14, 15, 16, 17, 18, 19, 20, 21, 22, 23, 24, 25, 26 or 27.

In Formula (I'), r is preferably 12, s is preferably 40 and q is preferably 0, 1, 2, or 3, thus resulting in a heteropolyoxometalate of the formula **[A**ₙ**]**^{m+}**(XM**_{q}**W**_{12-q}**O**₄₀**)**^{m-}.

In Formula (**II'**), when t = 6, q is preferably 0, resulting in a heteropolyoxometalate of the formula **[A**ₙ**]**^{m+}**[XW**₆**O**₂₄**]**^{m-}.

In Formula **(II')**, when t = 4, q is preferably 0, resulting in a heteropolyoxometalate of the formula **[A**ₙ**]**^{m+}**[XW**₄**O**₂₄**]**^{m-}.

In Formula (**III'**), p and q are preferably 0, resulting in a polyoxometalate of the formula **[A**ₙ**]**^{m+}**[W**₆**O**₁₉**]**^{m-}.

In Formula (**IV'**), q is preferably 0, 1, 2 or 3, and more preferably 0, resulting in a heteropolyoxometalate of the formula **[A**ₙ**]**^{m+}**[X**₂**W**₁₈**O**₆₂**]**^{m-}.

In Formula (**V'**), q is preferably 0, 1, 2, 3, and more preferably 0, resulting in a heteropolyoxometalate of the formula **[A**ₙ**]**^{m+}**[X**₅**W**₃₀**O**₁₁₀**]**^{m-}.

In a further preferred embodiment, the present invention relates to the use as described herein of (hetero)polyoxometalates of formulae **(I)**, **(II)**, **(III)**, **(IV)**, and **(V)**, wherein in formulae **(I)**, **(II)**, **(III)**, **(IV)**, and **(V)** Z = Mo. With o = 0, this embodiment covers (hetero)polyoxometalates of the following formulae **(I")**, **(II")**, **(III")**, **(IV")** and **(V")**:

**[A**ₙ**]**^{m+}**[XM**_{q}**Mo**_{r-q}**O**ₛ**]**^{m-} **(I")**,

wherein
m- is the negative charge of the heteropolyoxometalate anion,
m+ is the positive charge of the cation(s) A,
|m-| = |m+|
n is the number of cation(s) A required to provide the positive charge m+,
X is selected from P, Si, Ge, Al and B,
M is selected from Ti, V, Mn, Fe, Co, Ni, Zn and Cr,
r= 11 or 12,
s = 37, 38, 39 or 40,
when r = 11, q = 0, 1, 2, 3, 4, 5, 6, 7, 8 or 9,
when r = 12, q = 0, 1, 2, 3, 4, 5, 6, 7, 8, 9 or 10, and
when r = 12, s = 40, X = P and q is 0, 1, 2 or 3, M is not V;

**[A**ₙ**]**^{m+}**[XM**_{q}**Mo**_{t-q-o}**O**₂₄**]**^{m-} **(II")**,

wherein
m- is the negative charge of the heteropolyoxometalate anion,
m+ is the positive charge of the cation(s) A,
|m-| = |m+|
n is the number of cation(s) A required to provide the positive charge m+,
X is selected from P, Si, Ge, Al and B,
M is selected from Ti, V, Mn, Fe, Co, Ni, Zn and Cr,
t = 4 or 6,
when t = 6, q = 0, 1, 2, 3 or 4,
when t = 4, q = 0, 1 or 2, and
when t = 4 and X = P, q is 1, 2 or 3;

**[A**ₙ**]**^{m+}**[X**ₚ**M**_{q}**Mo**_{6-q-p}**O**₁₉**]**^{m-} **(III")**,

wherein
m- is the negative charge of the (hetero)polyoxometalate anion,
m+ is the positive charge of the cation(s) A,
|m-| = |m+|
n is the number of cation(s) A required to provide the positive charge m+, X is selected from P, Si, Ge, Al and B,
M is selected from Ti, V, Mn, Fe, Co, Ni, Zn and Cr,
p = 0 or 1, and
q = 0, 1, 2 or 3
when p = 0, M is not V;

**[A**ₙ**]**^{m+}**[X**₂**M**_{q}**Mo**_{18-q}**O**₆₂**]**^{m-} (IV"),

wherein
m- is the negative charge of the heteropolyoxometalate anion,
m+ is the positive charge of the cation(s) A,
|m-| = |m+|
n is the number of cation(s) A required to provide the positive charge m+,
X is selected from P, Si, Ge, Al and B,
M is selected from Ti, V, Mn, Fe, Co, Ni, Zn and Cr,
q = 0, 1, 2, 3, 4, 5, 6, 7, 8, 9, 10, 11, 12, 13, 14, 15 or 16, and
when X = P, q is 1, 2 or 3;

**[A**ₙ**]**^{m+}**[X**₅**M**_{q}**M**o_{30**-**q}**O**₁₁₀]^{m-} **(V")**,

wherein
m- is the negative charge of the heteropolyoxometalate anion,
m+ is the positive charge of the cation(s) A,
|m-| = |m+|
n is the number of cation(s) A required to provide the positive charge m+,
X is selected from P, Si, Ge, Al and B,
M is selected from Ti, V, Mn, Fe, Co, Ni, Zn and Cr,
q = 0, 1, 2, 3, 4, 5, 6, 7, 8, 9, 10, 11, 12, 13 ,14, 15, 16, 17, 18, 19, 20, 21, 22, 23, 24, 25, 26 or 27.

In Formula **(I")**, r is preferably 12, s is preferably 40 and q is preferably 0, 1, 2, or 3, thus resulting in a heteropolyoxometalate of the formula **[A**ₙ**]**^{m+}**[XM**_{q}**Mo**_{12-q}**O**₄₀**]**^{m-}.

In Formula (**II"**), when t = 6, q is preferably 0, resulting in a heteropolyoxometalate of the formula **[A**ₙ**]**^{m+}**[XMo**₆**O**₂₄**]**^{m-}.

In Formula (**II"**), when t = 4, q is preferably 0, resulting in a heteropolyoxometalate of the formula **[A**ₙ**]**^{m+}**[XMo**₄**O**₂₄**]**^{m-}.

In Formula **(III")**, p and q are preferably 0, resulting in a polyoxometalate of the formula **[A**ₙ**]**^{m+}**[Mo**₆**O**₁₉**]**^{m-}.

In Formula (**IV"**), q is preferably 0, 1, 2 or 3, and more preferably 0, resulting in a heteropolyoxometalate of the formula **[A**ₙ**]**^{m+}**[X**₂**Mo**₁₈**O**₆₂**]**^{m-}.

In Formula (**V"**), q is preferably 0, 1, 2, 3, and more preferably 0, resulting in a heteropolyoxometalate of the formula **[A**ₙ**]**^{m+}**[X**₅**Mo**₃₀**O**₁₁₀**]**^{m-}.

In a further preferred embodiment, the present invention relates to the use as described herein of (hetero)polyoxometalates of formulae **(I)**, **(II)**, **(III)**, **(IV)**, and **(V)**, wherein in formulae **(I)**, **(II)**, **(III)**, **(IV)**, and **(V)** Z = W and Z' = Mo and o = 1, thus covering (hetero)polyoxometalates of the following formulae **(I"')**, **(II"')**, **(III"')**, **(IV"')** and **(V"')**:

**[A**ₙ**]**^{m+}**[XM**_{q}**W**_{r-q-1}**MoO**ₛ**]**^{m-} **(I"')**,

wherein
m- is the negative charge of the heteropolyoxometalate anion,
m+ is the positive charge of the cation(s) A,
|m-| = |m+|
n is the number of cation(s) A required to provide the positive charge m+,
X is selected from P, Si, Ge, Al and B,
M is selected from Ti, V, Mn, Fe, Co, Ni, Zn and Cr,
r = 11 or 12,
s = 37, 38, 39 or 40,
when r = 11, q = 0, 1, 2, 3, 4, 5, 6, 7, 8 or 9,
when r = 12, q = 0, 1, 2, 3, 4, 5, 6, 7, 8, 9 or 10, and
when r = 12, s = 40, X = P and q is 0, 1, 2 or 3, M is not V;

**[A**ₙ**]**^{m+}**[XM**_{q}**W**_{t-q-1}**MoO**₂₄**]**^{m-} **(II'")**,

wherein
m- is the negative charge of the heteropolyoxometalate anion,
m+ is the positive charge of the cation(s) A,
|m-| = |m+|
n is the number of cation(s) A required to provide the positive charge m+,
X is selected from P, Si, Ge, Al and B,
M is selected from Ti, V, Mn, Fe, Co, Ni, Zn and Cr,
t = 4 or 6,
when t = 6, q = 0, 1, 2, 3 or 4,
when t = 4, q = 0, 1 or 2, and
when t = 4 and X = P, q is 1, 2 or 3;

**[A**ₙ**]**^{m+}**[X**ₚ**M**_{q}**W**_{5-q-p}**MoO**₁₉**]**^{m-} **(**III**"')**,

wherein
m- is the negative charge of the (hetero)polyoxometalate anion,
m+ is the positive charge of the cation(s) A,
|m-| = |m+|
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
o = 0 or 1,
p = 0 or 1, and
q = 0, 1, 2 or 3;
**[A**ₙ**]**^{m+}**[X**₂**M**_{q}**W**_{17-q}**MoO**₆₂**]**^{m-} **(IV'")**,

wherein
m- is the negative charge of the heteropolyoxometalate anion,
m+ is the positive charge of the cation(s) A,
|m-| = |m+|
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
o = 0 or 1,
q = 0, 1, 2, 3, 4, 5, 6, 7, 8, 9, 10, 11, 12, 13, 14, 15 or 16, and
when X = P, q is 1, 2 or 3;

**[A**ₙ**]**^{m+}**[X**₅**M**_{q}**W**_{29-q}**MoO**₁₁₀**]**^{m-} **(V"')**,

wherein
m- is the negative charge of the heteropolyoxometalate anion,
m+ is the positive charge of the cation(s) A,
|m-| = |m+|
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
o = 0 or 1,
q = 0, 1, 2, 3, 4, 5, 6, 7, 8, 9, 10, 11, 12, 13 ,14, 15, 16, 17, 18, 19, 20, 21, 22, 23, 24, 25, 26 or 27;

In Formula **(I"')**, r is preferably 12, s is preferably 40 and q is preferably 0, 1, 2, or 3, thus resulting in a heteropolyoxometalate of the formula **[A**ₙ**]**^{m+}**[XM**_{q}**W**_{11-q}**MoO**₄₀**]**^{m-}.

In Formula **(II"')**, when t = 6, q is preferably 0, resulting in a heteropolyoxometalate of the formula **[A**ₙ**]**^{m+}**[XW**₅**MoO**₂₄**]**^{m-}.

In Formula **(II"')**, when t = 4, q is preferably 0, resulting in a heteropolyoxometalate of the formula **[A**ₙ**]**^{m+}**[XW**₃**MoO**₂₄**]**^{m-}.

In Formula **(III"')**, p and q are preferably 0, resulting in a polyoxometalate of the formula **[A**ₙ**]**^{m+}**[W**₅**MoO**₁₉**]**^{m-}.

In Formula (**IV"'**), q is preferably 0, 1, 2 or 3, and more preferably 0, resulting in a heteropolyoxometalate of the formula **[A**ₙ**]**^{m+}**[X**₂**W**₁₇**MoO**₆₂**]**^{m-}.

In Formula (**V"'**), q is preferably 0, 1, 2, 3, and more preferably 0, resulting in a heteropolyoxometalate of the formula **[A**ₙ**]**^{m+}**[X**₅**W**₂₉**MoO**₁₁₀**]**^{m-}.

In a further preferred embodiment, the present invention relates to the use as described herein of (hetero)polyoxometalates of formulae **(I)**, **(II)**, **(III)**, **(IV)**, and **(V)**, wherein in formulae **(I)**, **(II)**, **(III)**, **(IV)**, and **(V)** X is selected from P, Si and Al.

It is also preferred that in formulae **(I')**, **(II')**, **(III')**, **(IV')**, and **(V')** X is selected from P, Si and Al.

It is furthermore preferred that in formulae **(I")**, **(II")**, **(III")**, **(IV")**, and **(V")** X is selected from P, Si and Al.

It is furthermore preferred that in formulae **(I"')**, **(II"')**, **(III"')**, **(IV"')**, and **(V"')** X is selected from P, Si and Al.

In a further preferred embodiment, the present invention relates to the use as described herein of (hetero)polyoxometalates of formulae (I), **(II)**, **(III)**, **(IV)**, and **(V)**, wherein in formulae **(I)**, **(II)**, **(III)**, **(IV)**, and **(V)** M is selected from Co, Ti and V.

It is also preferred that in formulae **(I')**, **(II')**, **(III')**, **(IV')**, and **(V')** M is selected from Co, Ti and V.

It is furthermore preferred that in formulae **(I")**, **(II")**, **(III")**, **(IV")**, and **(V")** M is selected from Co, Ti and V.

It is furthermore preferred that in formulae **(I"')**, **(II"')**, **(III"')**, **(IV"')**, and **(V"')** M is selected from Co, Ti and V.

In a further preferred embodiment, the present invention relates to the use as described herein of (hetero)polyoxometalates of formulae **(I)**, **(II)**, **(III)**, **(IV)**, and **(V)**, wherein in formulae **(I)**, **(II)**, **(III)**, **(IV)**, and **(V)** X = P or Si and M = V.

It is also preferred that in formulae **(I')**, **(II')**, **(III')**, **(IV')**, and **(V')** X = P or Si and M = V.

It is furthermore preferred that in formulae **(I")**, **(II")**, **(III")**, **(IV")**, and **(V")** X = P or Si and M = V.

It is furthermore preferred that in formulae **(I'")**, **(II"')**, **(III"')**, **(IV'")**, and **(V"')** X = P or Si and M = V.

In an alternative preferred embodiment, the present invention relates to the use as described herein of (hetero)polyoxometalates of formulae **(I)**, **(II)**, **(III)**, **(IV)**, and **(V)**, wherein in formulae **(I)**, **(II)**, **(III)**, **(IV)**, and **(V)** X = P or Si and M = Ti.

It is also preferred that in formulae **(I')**, **(II')**, **(III')**, **(IV')**, and **(V')** X = P or Si and M = Ti.

It is furthermore preferred that in formulae **(I")**, **(II")**, **(III")**, **(IV")**, and **(V")** X = P or Si and M = Ti.

It is furthermore preferred that in formulae **(I"')**, **(II"')**, **(III"')**, **(IV"')**, and **(V"')** X = P or Si and M = Ti.

In a further alternative preferred embodiment, the present invention relates to the use as described herein of (hetero)polyoxometalates of formulae **(I)**, **(II)**, **(III)**, **(IV)**, and **(V)**, wherein in formulae **(I)**, **(II)**, **(III)**, **(IV)**, and **(V)** X = P or Si and M = Co.

It is also preferred that in formulae **(I')**, **(II')**, **(III')**, **(IV')**, and **(V')** X = P or Si and M = Co.

It is furthermore preferred that in formulae **(I")**, **(II")**, **(III")**, **(IV")**, and **(V")** X = P or Si and M = Co.

It is furthermore preferred that in formulae **(I"')**, **(II"')**, **(III"')**, **(IV"')**, and **(V"')** X = P or Si and M = Co.

In a further alternative preferred embodiment, the present invention relates to the use as described herein of (hetero)polyoxometalates of formulae **(I)**, **(II)**, **(III)**, **(IV)**, and **(V)**, wherein in formulae **(I)**, **(II)**, **(III)**, **(IV)**, and **(V) (I)**, **(II)**, **(III)**, **(IV)**, and **(V)** X = Al and M = Co, V or Ti.

It is also preferred that in formulae **(I')**, **(II')**, **(III')**, **(IV')**, and **(V')** X = Al and M = Co, V or Ti.

It is furthermore preferred that in formulae **(I")**, **(II")**, **(III")**, **(IV")**, and **(V")** X = Al and M = Co, V or Ti.

It is furthermore preferred that in formulae **(I"')**, **(II"')**, **(III"')**, **(IV"')**, and **(V"')** X = Al and M = Co, V or Ti.

Particularly preferred (hetero)polyoxometalates for use in simultaneously imparting antimicrobial properties to a surface of a substrate and reducing, preferably inhibiting, the growth of a biofilm on the surface of the substrate are the following species:
Aₙ[SiVW₁₁O₄₀], Aₙ[SiV₂W₁₀O₄₀], Aₙ[SiV₃W₉O₄₀], Aₙ[SiV₄W₈O₄₀], Aₙ[SiVMo₁₁O₄₀], Aₙ[SiV₂Mo₁₀O₄₀], Aₙ[SiV₃Mo₉O₄₀], Aₙ[SiV₄Mo₈O₄₀], Aₙ[PV₆Mo₆O₄₀], Aₙ[PV₅Mo₇O₄₀], Aₙ([PV₈Mo₄O₄₀], Aₙ[PCoW₁₁O₃₉], Aₙ[AlVW₁₁O₄₀], Aₙ[SiVW₁₀MoO₄₀], based on heteropolyoxometalates of Formula (I);
Aₙ[PW₆O₂₄], Aₙ[PMo₆O₂₄], Aₙ[SiW₆O₂₄], Aₙ[SiMo₆O₂₄], Aₙ[AlW₆O₂₄], Aₙ[AlMo₆O₂₄], Aₙ[SiW₄O₂₄], Aₙ[SiMo₄O₂₄], Aₙ[AlW₄O₂₄], Aₙ[AlMo₄O₂₄], based on heteropolyoxometalates of Formula (II);
Aₙ[W₆O₁₉] and Aₙ[Mo₆O₁₉], based on polyoxometalates of Formula (III);
Aₙ[Si₂W₁₈O₆₂], Aₙ[Si₂Mo₁₈O₆₂], based on heteropolyoxometalates of Formula (IV);
Aₙ[P₅W₃₀O₁₁₀], Aₙ[Si₅W₃₀O₁₁₀], Aₙ[Al₅W₃₀O₁₁₀], Aₙ[P₅Mo₃₀O₁₁₀], Aₙ[Si₅Mo₃₀O₁₁₀], Aₙ[Al₅Mo₃₀O₁₁₀], based on heteropolyoxometalates of Formula (V).

It is understood that in these species, the cation(s) A is/are selected such that in the (hetero)polyoxometalate the charge is zero. For achieving this either the number and/or charge of cation(s) A can be altered and/or the charge is balanced by one or more further cations, e.g. selected from the ammonium ion (NHa⁺), alkaline metal ions such as Li⁺, Na⁺, K⁺, Rb⁺, Cs⁺, and alkaline earth metal ions such as Be²⁺, Mg²⁺, Ca²⁺, Sr²⁺, Ba²⁺.

Furthermore, it is possible that the (hetero)polyoxometalate anions comprise neutral ligands such as, e.g., H₂O, leading to structures such as, e.g., Aₙ[AlMn(H₂O)W₁₁O₃₀]

In a further preferred embodiment of the present invention, in the (hetero)polyoxometalate the quaternary ammonium cation is of Formula (VI), the quaternary phosphonium cation is of Formula (VII) and the tertiary sulfonium cation is of Formula (VIII)

**R¹R²R³R⁴N⁺** **(VI)**

**R¹R²R³R⁴P⁺** **(VII)**

**R¹R²R³S⁺** **(VIII)**

wherein residues R¹, R², R³ and R⁴ are independently selected from the group consisting of C1 to C80 hydrocarbons, and polymers, and optionally at least two of the residues R¹, R², R³ and, if present, R⁴ are part of a ring or form a ring together with the nitrogen, phosphor or sulfur atom.

These cations have antimicrobial properties contributing to the antimicrobial properties of the overall (hetero)polyoxometalate species and to its ability to reduce the biofilm growth on the surface of a substrate.

The presence of a quaternary ammonium cation of Formula (VI), a quaternary phosphonium cation of Formula (VII) or a tertiary sulfonium cation of Formula (VIII) also renders the (hetero)polyoxometalates as described herein less soluble or even insoluble in water. Preferably, the solubility of the (hetero)polyoxometalates of the present invention is below 10 mg/l, preferably below 1 mg/ml in water at 20°C, more preferably below 0.1 mg/ml water, in particular below 0.01 mg/ml water.

The lower solubility or even insolubility for aqueous liquids also contributes to the long term efficacy of the heteropolyoxometalate as described herein regarding the reduction of the biofilm growth on the surface of a substrate and antimicrobial activity since they are not washed out the substrate over time upon exposure to water or moisture.

The C1 to C80 hydrocarbons encompass branched or straight, saturated or unsaturated, substituted or unsubstituted alkyl groups, aryl groups or heteroaryl groups. These residues thus can also be waxes or wax-like.

Examples for these C1 to C80 hydrocarbons include methyl, ethyl, propyl, butyl, pentyl, hexyl, heptyl, octyl, nonyl, decyl, undecyl, dodecyl, tridecyl, tetradecyl, pentadecyl, hexadecyl, heptadecy, octadecyl, nonadecyl, eicosanyl, C21 alkyl, C22 alkyl, C23 alkyl, C24 alkyl, C25 alkyl, C26 alkyl, C27 alkyl, C28 alkyl, C29 alkyl, C30 alkyl, C31 alkyl, C32 alkyl, C33 alkyl, C34 alkyl, C35 alkyl, C36 alkyl, C37 alkyl, C39 alkyl, C40 alkyl, C41 alkyl, C42 alkyl, C43 alkyl, C44 alkyl, C45 alkyl, C46 alkyl, C47 alkyl, C48 alkyl, C49 alkyl, C50 alkyl, C51 alkyl, C52 alkyl, C53 alkyl, C54 alkyl, C55 alkyl, C56 alkyl, C57 alkyl, C58 alkyl, C59 alkyl, C60 alkyl, C61 alkyl C62 alkyl, C63 alkyl, C64 alkyl, C65 alkyl, C66 alkyl, C67 alkyl, C68 alkyl, C69 alkyl, C70 alkyl, C71 alkyl, C72 alkyl, C73 alkyl, C74 alkyl, C75 alkyl, C75 alkyl, C76 alkyl, C77 alkyl, C78 alkyl, C79 alkyl, and C80 alkyl residues. These C1 to C80 hydrocarbons are preferably straight.

Preferred are C1 to C60 hydrocarbons including methyl, ethyl, propyl, butyl, pentyl, hexyl, heptyl, octyl, nonyl, decyl, undecyl, dodecyl, tridecyl, tetradecyl, pentadecyl, hexadecyl, heptadecy, octadecyl, nonadecyl, eicosanyl, C21 alkyl, C22 alkyl, C23 alkyl, C24 alkyl, C25 alkyl, C26 alkyl, C27 alkyl, C28 alkyl, C29 alkyl, C30 alkyl, C31 alkyl, C32 alkyl, C33 alkyl, C34 alkyl, C35 alkyl, C36 alkyl, C37 alkyl, C39 alkyl, C40 alkyl, C41 alkyl, C42 alkyl, C43 alkyl, C44 alkyl, C45 alkyl, C46 alkyl, C47 alkyl, C48 alkyl, C49 alkyl, C50 alkyl, C51 alkyl, C52 alkyl, C53 alkyl, C54 alkyl, C55 alkyl, C56 alkyl, C57 alkyl, C58 alkyl, C59 alkyl and C60 alkyl residues. These C1 to C60 hydrocarbons are preferably straight.

More preferred are C1 to C40 hydrocarbons including methyl, ethyl, propyl, butyl, pentyl, hexyl, heptyl, octyl, nonyl, decyl, undecyl, dodecyl, tridecyl, tetradecyl, pentadecyl, hexadecyl, heptadecy, octadecyl, nonadecyl, eicosanyl, C21 alkyl, C22 alkyl, C23 alkyl, C24 alkyl, C25 alkyl, C26 alkyl, C27 alkyl, C28 alkyl, C29 alkyl, C30 alkyl, C31 alkyl, C32 alkyl, C33 alkyl, C34 alkyl, C35 alkyl, C36 alkyl, C37 alkyl, C39 alkyl and C40 alkyl residues. These C1 to C40 hydrocarbons are preferably straight.

More preferred are C1 to C30 hydrocarbons including methyl, ethyl, propyl, butyl, pentyl, hexyl, heptyl, octyl, nonyl, decyl, undecyl, dodecyl, tridecyl, tetradecyl, pentadecyl, hexadecyl, heptadecy, octadecyl, nonadecyl, eicosanyl, C21 alkyl, C22 alkyl, C23 alkyl, C24 alkyl, C25 alkyl, C26 alkyl, C27 alkyl, C28 alkyl, C29 alkyl, and C30 alkyl residues. These C1 to C30 hydrocarbons are preferably straight.

More preferred are C2 to C25 hydrocarbons including ethyl, propyl, butyl, pentyl, hexyl, heptyl, octyl, nonyl, decyl, undecyl, dodecyl, tridecyl, tetradecyl, pentadecyl, hexadecyl, heptadecy, octadecyl, nonadecyl, eicosanyl, C21 alkyl, C22 alkyl, C23 alkyl, C24 alkyl, and C25 alkyl. These C2 to C25 are preferably straight.

Particularly preferred are C4 to C20 hydrocarbons including butyl, propyl, hexyl, heptyl, octyl, nonyl, decyl, undecyl, dodecyl, tridecyl, tetradecyl, pentadecyl, hexadecyl, heptadecy, octadecyl, nonadecyl, and eicosanyl residues. These C4 to C20 hydrocarbons are preferably straight.

In general, it is more preferable that at least one of R¹, R², R³, and, if present, R⁴ is a hydrocarbon with at least 4 carbons, such as e.g. butyl, pentyl, hexyl etc. It is more preferable that at least two of R¹, R², R³, and, if present, R⁴ is a hydrocarbon with at least 4 carbons, such as e.g. butyl, pentyl, hexyl etc..

Suitable polymers include, for example, polymers comprising cationic side chains, such as phosphonium-containing cationic poly(styrene) polymers, hydroxy exchange membranes comprising quaternary ammonium hydroxide or quaternary phosphonium hydroxide functional groups, poly(vinylamine) derivatives as described for example in EP 0 580 078 A1, polymeric phosphonium ionomers, as described for example in WO 94/10214, poly(alkyl- and aryl)p-phenoxy-phenylsulfonium salts, poly(acrylamide-co-diallyl-dimethylammonium) and poly(diallyldimethylammonium).

The ring formed together with the nitrogen, phosphor or sulfur atom may, for example, be a three, four, five, six or seven-membered ring which may be saturated or unsaturated and which may contain one or more further heteroatoms, such as oxygen, nitrogen, phosphor or sulfur atoms. If the ring is unsaturated and if a double bond is present at the nitrogen, phosphor or sulfur atom of the ammonium, phosphonium or sulfonium cation, then one of R¹, R² R³ or R⁴ may be absent. Examples of suitable rings include aziridinium, thiiranium, azetidinium, thietium, pyrrolidinium, tetrathydrothiophenium, pyrrolium, thiophenium, piperidinium, tetrahydrothiopyranium, pyridinium, thiopyrylium, hexamethyleniminium, hexamethylensulfidium, azatropilidenium, thiotropilidenium, pyrazolium, imidazolium, benzimidazolium, imidazolinium, indolium, chinolinium, isochinolinium, purinium, pyrimidinium, oxazolium, thiazolium, thiazinium, triazines, thiazoles, thiazolines, piperidines, oxazolidines, oxazolidones, guanine derivatives, cytosine derivatives, adenine derivatives, thymine derivatives, cyclic amino-acid and peptide derivatives, cyclic biguanide derivatives, cyclic guanidine derivatives, as well as the phosphorous analogs of these ring systems.

The rings may be substituted by one or more hydrocarbon residues, in particular C₁ to C₁₂ alkyl or aryl (in particular phenyl) C₁ to C₆ alkyl residues. Suitable cations containing a ring are for example 1-butyl-3-methylimidazolium, 1-butyl-2,3-dimetylimidazolium, 1-methyl-3-octylimidazolium, 1-hexadecyl-3-methylimidazolium, 1,3-didecyl-3-methylimidazolium and 1-benzyl-3-methylimidazolium.

Preferred quaternary ammonium cations are e.g. tetramethylammonium, tetraethylammonium, tetrapropylammonium, tetrabutylammonium, tetrapentylammonium tetrahexylammonium, tetraheptylammonium tetraoctylammonium cation, tetranonylammonium cation, tetradecylammonium cation, tetraundecylammonium cation, tetradodecylammonium cation, tetratridecylammonium cation, tetratetradecylammonium cation, tetrapentadecylammonium cation, methyltributylammonium cation, methyltripentylammonium cation, methyltrihexylammonium cation, methyltriheptylammonium cation, methyltrioctylammonium cation, methyltrinonylammonium cation, methyltridecylammonium cation, methyltriundecylammonium cation, methyltridodecylammonium cation, methyltritridecylammonium cation, methyltritetradecylammonium cation, tributylhexylammonium cation, tributylheptylammonium cation, tributyloctylammonium cation, tributylnonylammonium cation, tributyldecylammonium cation, tributylundecylammonium cation, tributyldodecylammonium cation, tributyltridecylammonium cation, tributyltetradecylammonium cation, tributylpentadecylammonium cation, tributylhexadecylammonium cation, trihexyltetradecylammonium cation and trihexylhexadecylammonium cation, in particular tetrabutylammonium cation, tetrahexylammonium cation, methyltrioctylammonium cation, tributyltetradecylammonium cation.

Preferred quaternary phosphonium cations are e.g. tetrapropylphosphonium cation, tetrabutylphosphonium cation, tetrapentylphosphonium cation, tetrahexylphosphonium cation, tetraheptylphosphonium cation, tetraoctylphosphonium cation, tetraoctylphosphonium cation, tetranonylphosphonium cation, tetradecylphosphonium cation, tetraundecylphosphonium cation, tetradodecylphosphonium cation, tetratridecylphospphonium cation, tetratetradecylphosphonium cation, methyltrioctylphosphonium cation, tributyltetradecylphosphonium cation, tributyldodecylphosphonium cation, trihexyltetradecylphosphonium cation, trihexylhexadecylphosphonium cation in particular tetrabutylphosphonium cation, tetrahexylphosphonium cation, methyltributylphosphonium cation, methyltripentylphosphonium cation, methyltrihexylphosphonium cation, methyltriheptylphosphonium cation, methyltrioctylphosphonium cation, methyltrinonylphosphonium cation, methyltridecylphosphonium cation, tributylpentylphosphonium cation, tributylhexylphosphonium cation, tributylheptylphosphonium cation, tributyloctylphosphonium cation, tributylnonylphosphonium cation, tributyldecylphosphonium cation, tributylundecylphosphonium cation, tributyldodecylphosphonium cation, tributyltridecylphosphonium cation, tributyltetradecylphosphonium cation, tributylpentadecylphosphonium cation, tributylhexadecylphosphonium cation, trihexylheptylphosphonium cation, trihexyloctylphosphonium cation, trihexylnonylphosphonium cation, trihexyldecylphosphonium cation, trihexylundecylphosphonium cation, trihexyldodecylphosphonium cation, trihexyltridecylphosphonium cation, trihexyltetradecylphosphonium cation, trihexylpentadecylphosphonium cation and trihexylhexadecylphosphonium cation.

Preferred tertiary sulfonium cations are e.g. tributylsulfonium cation, tripentylsulfonium cation, trihexylsulfonium cation, triheptylsulfonium cation, trioctylsulfonium cation, methyldioctylsulfonium cation and dibutyltetradecylsulfonium cation, in particular tributylsulfonium cation and trihexylsulfonium cation.

Further suitable ammonium, phosphonium and sulfonium cations and the preparation thereof are known in the art.

In a preferred embodiment of the present invention, in the (hetero)polyoxometalate A comprises at least one cation selected from the group consisting of quaternary ammonium cations and quaternary phosphonium cations.

In a more preferred embodiment of the present invention, in the (hetero)polyoxometalate A comprises at least one, preferably at least two quaternary ammonium cations.

In an alternative more preferred embodiment of the present invention, in the (hetero)polyoxometalate A comprises at least one, preferably at least two quaternary phosphonium cations.

Particularly preferred (hetero)polyoxometalates are e.g.:
[(CH₃(CH₂)₃)₄N]₅[SiVW₁₁O₄₀], [(CH₃(CH₂)₃)₄N]₆[SiV₂W₁₀O₄₀], [(CH₃(CH₂)₅)₄N]₇[SiV₃W₉O₄₀], [(CH₃(CH₂)₃)₃P(C₁₄H₂₉)]₅[SiVW₁₁O₄₀], [(CH₃(CH₂)₃)₃P(C₁₄H₂₉)]₆[SiV₂W₁₀O₄₀] [(CH₃(CH₂)₃)₃P(C₁₄H₂₉)]₇[SiV₃W₉O₄₀], [(CH₃(CH₂)₃)₃P(C₁₄H₂₉)]₈[SiV₄W₈O₄₀], [(CH₃(CH₂)₃)₃P(C₁₄H₂₉)]₈[SiV₄Mo₈O₄₀], [(CH₃(CH₂)₇)₄N]₅[SiVMo₁₁O₄₀], [(CH₃(CH₂)₇)₃N(CH₃)]₆[SiV₂Mo₁₀O₄₀], [(CH₃(CH₂)₃)₃P((CH₂)₁₃CH₃)]₇[SiV₃Mo₉O₄₀], [(CH₃(CH₂)₃)₄N]₁₁[PV₈Mo₄O₄₀], [(CH₃(CH₂)₇)₃N(CH₃)]₅[PCoW₁₁O₃₉], [(CH₃(CH₂)₃)₃P((CH₂)₁₃CH₃)]₆[AlVW₁₁O₄₀], [(CH₃(CH₂)₃)₄N]₅[SiVW₁₀MoO₄₀], [(CH₃(CH₂)₃)₃P(C₁₄H₂₉)]₉[PV₆Mo₆O₄₀], [(CH₃(CH₂)₃)₃P(C₁₄H₂₉)]₈[PV₅Mo₇O₄₀], based on (hetero)polyoxometalates of Formula (I);
[(CH₃(CH₂)₃)₄N]₇[PW₆O₂₄], [(CH₃(CH₂)₇)₄N]₄[PMo₆O₂₄], [(CH₃(CH₂)₇)₃N(CH₃)]₈[SiW₆O₂₄], [(CH₃(CH₂)₃)₃P((CH₂)₁₃CH₃)]₈[SiMo₆O₂₄], [(CH₃(CH₂)₃)₄N]₉[AlW₆O₂₄], [(CH₃(CH₂)₃)₄N]₉[AlMo₆O₂₄], [(CH₃(CH₂)₅)₄N]₂₀[SiW₄O₂₄], [(CH₃(CH₂)₇)₄N]₂₀[SiMo₄O₂₄], [(CH₃(CH₂)₇)₃N(CH₃)]₂₁[AlW₄O₂₄], [(CH₃(CH₂)₃)₃P((CH₂)₁₃CH₃)]₂₁[AlMo₄O₂₄], based on (hetero)polyoxometalates of Formula (II);
[(CH₃(CH₂)₃)₄N]₂[W₆O₁₉] and [(CH₃(CH₂)₃)₃P((CH₂)₁₃CH₃)]₂[Mo₆O₁₉], based on (hetero)polyoxometalates of Formula (III);
[(CH₃(CH₂)₇)₃N(CH₃)]₈[Si₂W₁₈O₆₂], [(CH₃(CH₂)₃)₃P((CH₂)₁₃CH₃)]₈[Si₂Mo₁₈O₆₂], based on (hetero)polyoxometalates of Formula (IV);
[(CH₃(CH₂)₃)₄N]₁₅[P₅W₃₀O₁₁₀], [(CH₃(CH₂)₇)₃N(CH₃)]₂₀[Si₅W₃₀O₁₁₀], [(CH₃(CH₂)₅)₄N]₁₅[P₅Mo₃₀O₁₁₀], [(CH₃(CH₂)₅)₄N]₂₀[Si₅Mo₃₀O₁₁₀], based on (hetero)polyoxometalates of Formula (V).

These compounds are particularly suitable for simultaneously imparting antimicrobial properties to a surface of a substrate and reducing, particularly inhibiting, the growth of a biofilm on the surface of the substrate because they are particularly suitable for activating molecular oxygen and producing ROS, thereby reducing, preferably inhibiting, the growth of biofilm on a substrate surface and maintaining the microbial efficacy of the heteropolyoxometalates on a long term time scale (at least 2 weeks month, preferably at least 1 month, more preferably at least 1 year, even more preferably at least 3 years).

The (hetero)polyoxometalates of formulae **(I)**, **(II)**, **(III)**, **(IV)** and **(V)** and sub-formulae thereof as described herein can be prepared according to known processes. Often, heteropolyoxometalates can be prepared from minimally toxic compounds such as e.g. WO₄²⁻, MoO₄²⁻, PO₄³⁻, SiO₃²⁻VO₃- (see I.A. Weinstock et al., Selective Transition-Metal Catalysis of Oxygen Delignification Using Water-Soluble Salts of Polyoxometalate (POM) Anions, Part I. Chemical Principles and Process Concepts, Holzforschung 1998, 52, 304-310). General synthesis methods are also disclosed in EP 2 765 136 A1 and WO 2014/122225 A1. Syntheses of exemplary heteropolyoxometalates as disclosed herein are described in the examples.

The present invention furthermore relates to a substrate comprising a (hetero)polyoxometalate as defined above, or a mixture thereof, wherein the substrate is selected from the group consisting of a polymer body, a paint, a varnish, a coating, an ink, glass fibers or an inorganic oxide material.

A polymer body, as referred to herein, is any object comprising at least one polymer. The polymer body can e.g. be selected from the group consisting of: containers for storage of drinking water (e.g. in the beverage industry), waste water, or surface water; containers for waste decomposition; containers for water purification; heating systems; medical equipment; ship, boat, and roof coverings; roof tiles, indoor tiles, outdoor tiles; kitchen equipment; bathroom equipment; toilets, portable toilets; fibers and non-wovens; technical textiles, activewear fabrics; rain water sewers; exterior façades, elements of façades; tubing; furniture; water filters; air conditioning systems; air filters; safety protection masks; respirators; automotive air conditioning systems; humidifiers; disinfection sprays; air dryers; wall paints; packaging.

The term "paint", as used herein, refers to a liquid composition which upon administration to a substrate in a thin layer converts into solid, colored film. A paint, as referred to herein, also comprises lacquers. The term "paint" thus refers to the liquid composition before administration to a substrate and to the solid, colored film obtained after administration of the liquid paint composition to the substrate, and the skilled person will understand from the context, which state of the paint is meant. The color of the paint is achieved by colorants or pigments comprised in the paint.

The term "varnish", as used herein, refers to a liquid composition which upon administration to a substrate in a thin layer converts into a solid, colorless film. The term "varnish" thus refers to the liquid composition before administration to a substrate and to the solid, colorless film obtained after administration of the liquid varnish composition to the substrate, and the skilled person will understand from the context, which state of the varnish is meant.

The term "coating", as used herein, is any liquid composition that upon administration to a substrate in a thin layer converts into a solid film for decorative or protective purposes. The term "coating" thus refers to the liquid composition before administration to a substrate and to the solid film obtained after administration of the liquid coating composition to the substrate, and the skilled person will understand from the context, which state of the coating is meant.

The term "ink", as used herein, is a liquid composition containing pigments or dyes that upon administration to a substrate converts into a colored film for illustrating and/or lithographic purposes.

These paints, varnishes, coatings and inks typically comprise polymers selected from the group consisting of acrylate resins, polyurethane resins, silicon resins, polyester resins, alkyd resins, epoxy resins, phenolic resins, and urea or amine based resins.

In a preferred embodiment of the present invention, the substrate comprises a thermoplastic, an elastomer, a thermoplastic elastomer, a duroplast, or a mixture thereof.

The term "thermoplastic", as used herein, refers to a polymer that becomes pliable or moldable above a specific temperature and solidifies upon cooling. Examples for thermoplastic polymers include but are not limited to polyacrylates, acrylonitrile-butadiene-styrenes, polyamides such as nylon, polyacetic acid, polybenzimidazole, polycarbonate, polyether sulfone, polyetherether ketone, polyetherimide, polyethylene, polyphenylene oxide, polyphenylene sulfide, polypropylene, polystyrene, polyvinylchloriode, polyethyleneterephthalate, polyurethane, polyester and polytetrafluoroethylene (e.g. Teflon).

The term "elastomer", as used herein, refers to a polymer with viscoelasticity (having both viscosity and elasticity). Examples for elastomers include but are not limited to unsaturated rubbers such as natural polyisoprene (natural rubber), synthetic polyisoprene, polybutadiene, chloroprene rubber, butyl rubber, styrene-butadiene rubber, (hydrogenated) nitrile rubber, saturated rubbers such as ethylene propylene rubber, ethylene propylene diene rubber, epichlorohydrin rubber, polyacrylic rubber, silicone, silicone rubber, fluorosilicone rubber, fluoro- and perfluoroelastomers, and ethylene-vinyl acetate.

The term "thermoplastic elastomer", as used herein, refers to a class of copolymers or a physical mix of polymers which consists of materials with both thermoplastic and elastomeric properties. Examples for thermoplastic elastomers include but are not limited to styrenic block copolymers, polyolefin blends, elastomeric alloys, thermoplastic polyurethanes, thermoplastic copolyester, and thermoplastic polyamides.

The term "duroplast", as used herein, refers to a polymer which is no longer pliable after curing. Examples for duroplasts include but are not limited to aminoplasts, phenoplasts, epoxy resins, polyacrylates, polyurethanes, polyesters, urea formaldehyde resins, melamine formaldehyde resins, and phenol formaldehyde resins.

In a more preferred embodiment of the present invention, the substrate comprises a polymer selected from the group consisting of polypropylene, polyethylene, polyethyleneterephthalate, polyester, polyamide, polyurethane, polyacrylate, polycarbonate, polystyrene, polyimides, polymethacrylates, polyoxoalkylenes, poly(phenylene oxides), polyvinylesters, polyvinylethers, polyvinylidene chloride, acrylonitrile-butadiene-styrene, natural and synthetic polyisoprene, polybutadiene, chloroprene rubber, styrene-butadiene rubber, tetrafluoroethylene, silicone, acrylate resins, polyurethane resins, silicone resins, polyester resins, alkyd resins, epoxy resins, phenolic resins, and urea or amine based resins, or a mixture thereof.

In a further preferred embodiment, the substrate comprises the (hetero)polyoxometalate or the mixture thereof in an amount of 0.01 to 90 wt.-%, based on the total weight of the substrate, more preferably in an amount of 0.02 to 85 wt.-%, 0.05 to 80 wt.-%, 0.1 to 75 wt.-%, 0.2 to 70 wt.-%, 0.3 to 65 wt.-%, 0.4 to 60 wt.-%, 0.5 to 55 wt.-% and particularly preferably in an amount of 1 to 50 wt.-%, each based on the total weight of the substrate. The surface of a substrate wherein this amount of (hetero)polyoxometalate is present, is particularly suitable for imparting antimicrobial properties to the surface of the substrate and reduces, preferably inhibits, at the same time the growth of a biofilm on the surface of the substrate. Moreover, since the (hetero)polyoxometalates as described herein have a low solubility in water, they are not washed out of the substrate upon exposure to water and moisture which guarantees a long term efficacy of the (hetero)polyoxometalate in providing antimicrobial properties to the surface of the substrate and reducing, preferably inhibiting, the growth of a biofilm.

The (hetero)polyoxometalates as described herein can be incorporated into a substrate as described above, i.e. into a polymer body, a paint, a varnish or a coating, by means of techniques known to the person skilled in the art.

The incorporation of the (hetero)polyoxometalate into a polymer body can e.g. be achieved by mixing the (hetero)polyoxometalate with the polymer in order to obtain a dispersion or a compound of (hetero)polyoxometalate and polymer. Said dispersion or compound can then e.g. be submitted to injection molding or extrusion for forming the polymer body.

Alternatively, the (hetero)polyoxometalate can be mixed with the monomers before polymerization is carried out. After polymerization, the resulting polymer can e.g. be submitted to compounding processes, pressureless processing techniques (e.g. casting, dipping, coating, foaming) or compression molding, rolling and calendaring, extrusion, blow molding or injection molding processes or drawing, thermoforming or printing for forming the polymer body.

The polymer body as described herein can also be obtained by drylaid, airlaid, spunlaid/meltblown or wetlaid processes, in particular if the polymer body is for use as fiber material or non-woven material.

The incorporation of the (hetero)polyoxometalate into a paint can e.g. be achieved by mixing the (hetero)polyoxometalate, which e.g. can be used as a powder, with the liquid paint composition in order to obtain a suspension of the (hetero)polyoxometalate and the liquid paint composition. Said suspension can be stored before application of the paint in its intended use.

The incorporation of the (hetero)polyoxometalate into a varnish can e.g. be achieved by mixing the (hetero)polyoxometalate, which e.g. can be used as a powder, with the liquid varnish composition in order to obtain a suspension of the (hetero)polyoxometalate and the liquid varnish. Said suspension can be stored before application of the varnish in its intended use.

The incorporation of the (hetero)polyoxometalate into a liquid coating composition can e.g. be achieved by mixing the (hetero)polyoxometalate, which e.g. can be used as a powder, with a coating solution in order to obtain a suspension of the (hetero)polyoxometalate and the liquid coating solution. Said suspension can be stored before usage, i.e. application of the coating solution in its intended use.

The substrate comprising the (hetero)polyoxometalate as described herein can be used for self-cleaning, stripping, disinfecting, self-sanitizing, biocidal, antimicrobial, and/or deodorizing purposes and/or for the decomposition and/or degradation of organic materials.

In particular, the substrate comprising the (hetero)polyoxometalate as described herein can be used for the disinfection of an aqueous media by means of incorporating the substrate into an aqueous media such as waste water, surface water, drinking water etc.. The (hetero)polyoxometalate comprised in the substrate then activates molecular oxygen and/or hydrogen peroxide comprised in the aqueous media, thereby generating reactive oxygen species (ROS) such as the hyperoxide anion (O₂⁻), which are emitted from the surface of the substrate being surrounded by the aqueous media, thereby evolving an antimicrobial and disinfecting effect in the surrounding aqueous media. The oxidized (hetero)polyoxometalate in the surface of the substrate, in particular the cationic moiety thereof, reacts with the negatively charged cell membrane of microorganisms contained in the surrounding aqueous media by means of electron transfer, thereby regenerating the heteropolyoxometalate, which subsequently activates molecular oxygen comprised in the aqueous medium. The substrates for this application include filter materials, non-wovens, polymers, glas fibers or inorganic oxide materials comprising the (hetero)polyoxometalates of the present invention, and which are provided with a large surface.

### Examples

### Example 1: Synthesis of [(C₄H₉)₃P(C₁₄H₂₉)]₅[SiVW₁₁O₄₀] (It-1)

K₈[α-SiW₁₁O₃₉]×13H₂O was dissolved in hot water under stirring. After cooling, the pH was adjusted to 5.8 with HCl. NaVO₃ solution was slowly added under stirring, while the pH was adjusted to 5.8 with HCl, and stirred for 5 min. Next, the pH was adjusted to 2.0 using HCl and the solution was stirred at the same pH for 45 min. The resulting solution was filtered and TBTDP-Cl ([(C₄H₉)₃P(C₁₄H₂₉)]Cl) was added. The yellow precipitate was separated by suction filtration, washed with H₂O and dried under vacuum.

### Example 2: Synthesis of [(C₄H₉)₃P(C₁₄H₂₉)]₇[SiV₃W₉O₄₀] (It-2)

NaVO₃ (0.7 g, 6.0 mmol) and 5.0 g of Na₁₀[α-SiW₉O₃₄]×xH₂O (2.0 mmol) were mixed as dry powders and added to 50 ml of H₂O at room temperature (25°C). The solution was stirred vigorously during 30 min and then 6 M HCL was added dropwise to bring the pH to 1.5, and a clear wine red solution developed. The resulting solution was filtered and 15.0 g of TBTDP-Cl ([(C₄H₉)₃P(C₁₄H₂₉)]Cl) was added. The orange gel was separated by removing the upper aqueous layer and washed 5 times with 50 ml of H₂O.

### Comparative Example 1: Synthesis of [(C₄H₉)₃P(C₁₄H₂₉)]₇[PVW₁₁O_{4O}] (It-4)

H₃PW₁₂O₄₀ was dissolved in H₂O and solid Li₂CO₃ was added to pH 4.9. The solution of NaVO₃ was added with stirring. HCl was added dropwise to pH 2 and the solution was heated to 60 °C for 10 min before returning it to room T. Additional HCl was added to bring the mixture back to pH 2 and the solution was reheated to 60 °C. The resulting solution was filtered and TBTDP-Cl ([(C₄H₉)₃P(C₁₄H₂₉)]Cl) was added. The yellow precipitate was separated by suction filtration, washed with H₂O and dried under vacuum.

### Example 3: Antimicrobial surface activity

The compounds It-1, It-2 and It-4, respectively obtained in Examples 1 and 2 and Comparative Example 1, were used in the preparation of test pieces for the determination of the antimicrobial surface activity of these compounds.

Samples of the compounds It-1, It-2, and It-4 were respectively incorporated into an acrylate-based composition (said acrylate composition being designated in the following as "A1220", consisting of 57% (w/w) WorléeCryl (WorléeChemie), 14% (w/w) Desmodur N75 BA (Bayer) and 29% (w/w) acrylate thinner (Gräsolin), each based on the total weight of the acrylate composition), resulting in (hetero)polyoxometalate-functionalized compositions with a 30% (w/w) loading of It-1 (Sample 1), It-2 (Sample 2), and It-4 (Sample 3), based on the total weight of the composition.

Polypropylene (PP) pieces (ca. 5 x 5 cm) were respectively coated with a layer of the hetero)polyoxometalate-functionalized compositions according to Sample 1, Sample 2 and Sample 3, and the resulting coated pieces were cured in the open air. For each sample, 6 polypropylene test pieces were prepared.

6 further polypropylene pieces were coated with a neutral layer of A1220 (i.e. not comprising a (hetero)polyoxometalate) as the blind sample (BS).

The test microorganism (Staphylococcus aureus, DSMZ No. 799) was prepared by growth in a liquid culture medium (trypticase soy broth, TSB). The suspension of test microorganism was standardized by dilution in a nutrient broth.

The surfaces of Samples 1 to 3 and the blind sample (BS) were inoculated with microorganism and then the microbial inoculum was covered with a thin, sterile film in order to spread the inoculum, prevent it from evaporating and to ensure close contact with the antimicrobial surface. Microbial concentrations of Samples 1 to 3 and the blind sample (BS) were determined at "time zero" (t = 0) by elution, followed by dilution and plating. Samples 1 to 4 were then allowed to incubate undisturbed in a humid environment for 24 hours at 36 °C and 90% relative humidity. After incubation, microbial concentrations were determined by elution, followed by dilution and plating.

The antimicrobial activity value (R) of Samples 1 to 3 was determined via:
R = log (B/A) - log (C/A), wherein:
   A is the average of the CFU value of the untreated surface, after t = 0 hrs,
   B is the average of the CFU value of the untreated surface, after 24 hrs incubation,
   C is the average of the CFU value of the active surface, after 24 hrs incubation,
   wherein CFU is the colony forming unit.

The results for Samples 1 to 3 and the blind sample (BS) are shown in Table 1 (based on the average of 6 test pieces for each sample).

**Table 1**

| **Sample #** | **Coating of PP** | **t = 0** | **t = 24 hrs** | **Antimicrobial activity value (R)** |
|---|---|---|---|---|
| | | **CFU/test surface** | | |
| 1 | 30% It-1 in A1220 | - | 17 | 5,44 |
| 2 | 30% It-2 in A1220 | - | < 10 | > 5,66 |
| 3 | 30% It-4 in A1220 | - | 410 | 4,05 |
| BS | neutral A1220 | 1,3 x 10⁷ | 4,6 x 10⁶ | - |

The surfaces of both Samples 1 to 2 showed a high antimicrobial activity. Particularly high killing rates were achieved with Sample 2 (It-2). In this sample, no microorganisms were detectable after 24 hours. Sample 1 (It-1) also showed a high value of antimicrobial activity. Compared to Samples 1 and 2, the antimicrobial activity of Sample 3 was significantly lower.

### Example 4: Reduction of the growth of biofilm

The compound It-2, obtained in Example 2, was used in the preparation of an heteropolyoxometalate-functionalized active coating consisting of the acrylic polymer composition A1220 as described above, and 10% (w/w) of It-2 (Sample 1), based on the total weight of the active coating.

2 polypropylene (PP) test pieces (2.5 x 5 cm) were prepared by coating one part of the test piece with the active coating according to Sample 1. The other part of the test piece remained free of active coating. 2 further polypropylene pieces without coating were used as the blind sample (BS).

The test microorganism (Staphylococcus aureus (S. aureus), DSMZ No. 799) was prepared by growth in a liquid culture medium (trypticase soy broth, TSB). The suspension of test microorganism was standardized by dilution in a nutrient broth. The polypropylene test pieces according to Sample 1 were immersed in the S. aureus containing nutrient solution in a petri dish, respectively.

The samples prepared above are summarized in the following Table 2:

**Table 2**

| **Sample #** | **Coating of PP** | **Description** |
|---|---|---|
| 1 | 10% It-2 in A1220 | 2 PP test pieces (2.5 x 5 cm), one part with active coating |
| BS | - | 2 PP test pieces (2.5 x 5 cm) without active coating |

After 1 week, one of the polypropylene test pieces of Sample 1 and of the blind sample (BS) was taken out of the petri dish, dried at 20°C, and submitted to SEM (scanning electron microscope) spectroscopy. The results after 1 week for Sample 1 comparing the coated part (left SEM image) with the uncoated part of the polypropylene test piece (right SEM image) are shown in Fig. 1a. The result for the blind sample (BS) as determined by SEM spectroscopy after 1 week is shown in Fig. 2a.

The nutrient solution in the petri dishes was replaced by a fresh nutrient solution containing the microorganism, and the polypropylene test pieces according to Sample 1 and the blind sample (BS) were allowed to rest for another week.

After 2 weeks in total, a further polypropylene test piece of Sample 1 and the blind sample (BS) was taken out of the petri dish, dried at 20°C, and submitted to SEM spectroscopy. The results after 2 weeks for Sample 1 comparing the coated part (left SEM image) with the uncoated part of the test piece (right SEM image) are shown in Fig. 1 b. The result for the blind sample (BS) as determined by SEM spectroscopy after 2 weeks is shown in Fig. 2b.

Fig. 1 a shows the growth of S. aureus biofilm on both the coated part of the test piece (left SEM image of Fig. 1 a) and the uncoated part of the test piece (right SEM image of Fig. 1 a) after 1 week. Fig. 1b no longer shows S. aureus biofilm on both the coated part of the test piece (left SEM image of Fig. 1 b) and the uncoated part of the test piece (right SEM image of Fig. 1b) after 2 weeks. The contamination shown in Fig. 1b is mold fungus.

Sample 1 thus shows a reduction of the growth of biofilm after 2 weeks exposure to a nutrient medium containing S. aureus microorganism. The effect of the active coating also spreads into the uncoated vicinity of the active coating.

The blind sample (BS) does not show this decrease in the biofilm growth after 2 weeks exposure to a nutrient medium containing S. aureus microorganism (compare Fig. 2a, taken after 1 week to Fig. 2b, taken after 2 weeks.

## Claims

1. Use of at least one (hetero)polyoxometalate for simultaneously imparting antimicrobial properties to a surface of a substrate and reducing the growth of a biofilm on the surface of the substrate, wherein the at least one (hetero)polyoxometalate is of the formula **(I)**, **(II)**, **(III)**, **(IV)** and/or **(V)**:
**[A**ₙ**]**^{m+}**[XM**_{q}**Z**_{r-q-o}**Z'**ₒ**O**ₛ**]**^{m-} **(I)**,
wherein
m- is the negative charge of the heteropolyoxometalate anion,
m+ is the positive charge of the cation(s) A,
|m-| = |m+|
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
r=11 or 12,
o = 0 or 1,
s = 37, 38, 39 or 40,
when r = 11, q = 0, 1, 2, 3, 4, 5, 6, 7, 8 or 9,
when r = 12, q = 0, 1, 2, 3, 4, 5, 6, 7, 8, 9 or 10, and
when r = 12, s = 40, X = P and q is 0, 1, 2 or 3, M is not V;
**[A**ₙ**]**^{m+}**[XM**_{q}**Z**_{t-q-o}**Z'**ₒ**O**₂₄**]**^{m-} **(II)**,
wherein
m- is the negative charge of the heteropolyoxometalate anion,
m+ is the positive charge of the cation(s) A,
|m-| = |m+|
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
t = 4 or 6,
o = 0 or 1,
when t = 6, q = 0, 1, 2, 3 or 4,
when t = 4, q = 0, 1 or 2, and
when t = 4 and X = P, q is 1, 2 or 3;
**[A**ₙ**]**^{m+}**[X**ₚ**M**_{q}**Z**_{6-q-p-o}**Z'**ₒ**O**₁₉**]**^{m-} **(III)**,
wherein
m- is the negative charge of the (hetero)polyoxometalate anion,
m+ is the positive charge of the cation(s) A,
|m-| = |m+|
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
o = 0 or 1,
p = 0 or 1, and
q = 0, 1, 2 or 3,
when p = 0, M is not V;
**[A**ₙ**]**^{m+}**[X**₂**M**_{q}**Z**_{18-q-o}**Z'**ₒ**O**₆₂**]**^{m-} **(IV)**,
wherein
m- is the negative charge of the heteropolyoxometalate anion,
m+ is the positive charge of the cation(s) A,
|m-| = |m+|
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
o = 0 or 1,
q = 0, 1, 2, 3, 4, 5, 6, 7, 8, 9, 10, 11, 12, 13, 14, 15 or 16, and
when X = P, q is 1, 2 or 3;
**[Aₙ]^{m+}[X₅M_{q}Z_{30-q-o}Z'ₒO₁₁₀]^{m-}** **(V)**,
wherein
m- is the negative charge of the heteropolyoxometalate anion,
m+ is the positive charge of the cation(s) A,
|m-| = |m+|
n is the number of cation(s) A required to provide the positive charge m+,
Z and Z' are independently selected from W and Mo,
X is selected from P, Si, Ge, Al and B,
M is selected from Ti, V, Mn, Fe, Co, Ni, Zn and Cr,
o = 0 or 1,
q = 0, 1, 2, 3, 4, 5, 6, 7, 8, 9, 10, 11, 12, 13 ,14, 15, 16, 17, 18, 19, 20, 21, 22, 23, 24, 25, 26 or 27;
wherein A is selected from one or more cations and comprises at least one cation selected from the group consisting of quaternary cations, quaternary phosphonium cations and tertiary sulfonium cations,
with the proviso that the heteropolyoxometalate is not [(CH₃(CH₂)₅)₄N]₇[SiV₃W₉O₄₀].

2. Use according to claim 1, wherein in formulae **(I)**, **(II)**, **(III)**, **(IV)**, and **(V)** Z = W.

3. Use according to claim 1, wherein in formulae **(I)**, **(II)**, **(III)**, **(IV)**, and **(V)** Z = Mo.

4. Use according to any of claims 1 to 3, wherein in formulae **(I)**, **(II)**, **(III)**, **(IV)**, and **(V)** X is selected from P, Si and Al.

5. Use according to any of claims 1 to 4, wherein in formulae **(I)**, **(II)**, **(III)**, **(IV)**, and **(V)** M is selected from Co, Ti and V.

6. Use according to any of claims 1 to 5, wherein in formulae **(I)**, **(II)**, **(III)**, **(IV)**, and **(V)** X = P or Si and M = V.

7. Use according to claims 1 to 5, wherein in formulae **(I)**, **(II)**, **(III)**, **(IV)**, and **(V)** X = P or Si and M = Ti.

8. Use according to claims 1 to 5, wherein in formulae **(I)**, **(II)**, **(III)**, **(IV)**, and **(V)** X = P or Si and M = Co.

9. Use according to claims 1 to 5, wherein in formulae **(I)**, **(II)**, **(III)**, **(IV)**, and **(V)** X = Al and M = Co, V or Ti.

10. Use according to any of the preceding claims, wherein in the (hetero)polyoxometalate the quaternary ammonium cation is of Formula (VI), the quaternary phosphonium cation is of Formula (VII) and the tertiary sulfonium cation is of Formula (VIII)
**R¹R²R³R⁴N⁺** **(VI)**
**R¹R²R³R⁴P⁺** **(VII)**
**R¹R²R³S**⁺ **(VIII)**
wherein residues R¹, R², R³ and R⁴ are independently selected from the group consisting of C1 to C80 hydrocarbons and polymers, and optionally at least two of the residues R¹, R², R³ and, if present, R⁴ are part of a ring or form a ring together with the nitrogen, phosphor or sulfur atom.

11. Use according to any of the preceding claims, wherein in the (hetero)polyoxometalate A comprises at least one cation selected from the group consisting of quaternary ammonium cations and quaternary phosphonium cations.

12. A substrate comprising a (hetero)polyoxometalate as defined in any of the preceding claims, or a mixture thereof, wherein the substrate is selected from the group consisting of a polymer body, a paint, a varnish, a coating, an ink, glass fibers and an inorganic oxide material.

13. Substrate according to claim 12, wherein the substrate further comprises a polymer selected from the group consisting of thermoplastics, elastomers, thermoplastic elastomers, and duroplasts, preferably selected from polypropylene, polyethylene, polyethyleneterephthalate, polyester, polyamide, polyurethane, polyacrylate, polycarbonate, polystyrene, polyimides, polymethacrylates, polyoxoalkylenes, poly(phenylene oxides), polyvinylesters, polyvinylethers, polyvinylidene chloride, acrylonitrile-butadiene-styrene, natural and synthetic polyisoprene, polybutadiene, chloroprene rubber, styrene-butadiene rubber, tetrafluoroethylene, silicone, acrylate resins, polyurethane resins, silicone resins, polyester resins, alkyd resins, epoxy resins, phenolic resins, and urea or amine based resins, or a mixture thereof.

14. Substrate according to claim 12 or 13, wherein the substrate comprises the (hetero)polyoxometalate in an amount of 0.01 to 90 wt.-%, based on the total weight of the substrate.

15. Substrate according to any of claims 12 to 14, wherein the substrate is a polymer body obtainable by compounding processes, pressureless processing techniques (e.g. casting, dipping, coating, foaming) or compression molding, rolling and calendaring, extrusion, blow molding or injection molding processes or drawing, thermoforming or printing or drylaid, airlaid, spunlaid/meltblown or wetlaid processes.

16. Use of a substrate as defined in claims 12 to 15 for self-cleaning, stripping, disinfecting, self-sanitizing, biocidal, antimicrobial, and/or deodorizing purposes and/or for the decomposition and/or degradation of organic materials.

17. Use of a substrate as defined in claims 12 to 15 for the disinfection of an aqueous medium.
